# EUROPEAN PATENT SPECIFICATION

(11) **EP 1 755 590 B1**
(45) Date of publication and mention of the grant of the patent: **24.08.2011**
(21) Application number: 05749635.8
(22) Date of filing: 17.05.2005
(51) Int. Cl.: A61K 31/402, A61K 31/4704, A61K 31/428, A61P 11/00

(54) **COMBINATION OF GLYCOPYRROLATE AND A BETA2 ADRENOCEPTOR AGONIST**
KOMBINATION AUS GLYCOPYRROLAT UND EINEM BETA2-ADRENOCEPTOR-AGONIST
COMBINAISON DE GLYCOPYRROLATE ET D'UN AGONISTE DE RECEPTEUR BETA2-ADRENERGIQUE

(30) Priority: 18.05.2004 GB 0411056
(43) Date of publication of application: 28.02.2007
(60) Divisional application: 10164976.2
(73) Proprietor: Novartis AG, 4056 Basel (CH); Novartis Pharma GmbH, 1230 Wien (AT)
(72) Inventor: COLLINGWOOD, Stephen Paul, Horsham, West Sussex RH12 5AB (GB)
(74) Representative: McLean, Craig Sutherland
(86) International application number: PCT/EP2005/005354
(87) International publication number: WO 2005/110402

(56) References cited:
- WO-A-00/75114
- WO-A-2004/016601
- US-A1- 2004 002 548
- CYDULKA R K ET AL: "EFFECTS OF COMBINED TREATMENT WITH GLYCOPYRROLATE AND ALBUTEROL IN ACUTE EXACERBATION OF CHRONIC OBSTRUCTIVE PULMONARY DISEASE" ANNALS OF EMERGENCY MEDICINE, LANSING, MI, US, vol. 25, April 1995 (1995-04), pages 470-473, XP000979833 ISSN: 0196-0644
- TZELEPIS G ET AL: "COMPARISON OF NEBULIZED GLYCOPYRROLATE AND METAPROTERENOL IN CHRONIC OBSTRUCTRIVE PULMONARY DISEASE" EUROPEAN RESPIRATORY JOURNAL, MUNKSGAARD INTERNATIONAL PUBLISHERS, COPENHAGEN, DK, vol. 9, no. 8, 1996, pages 100-103, XP000979863 ISSN: 0903-1936
- CALVO G M ET AL: "IS IT USEFUL TO ADD AN ANTICHOLINERGIC TREATMENT TO BETA2-ADRENERGIC MEDICATION IN ACUTE ASTHMA ATTACK" JOURNAL OF INVESTIGATIONAL ALLERGOLOGY AND CLINICAL IMMUNOLOGY, BARCELONA, ES, vol. 8, no. 1, January 1998 (1998-01), pages 30-34, XP009018610 ISSN: 1018-9068
- NOORD VAN J A ET AL: "LONG-TERM TREATMENT OF CHRONIC OBSTRUCTIVE PULMONARY DISEASE WITH SALMETEROL AND THE ADDITIVE EFFECT OF IPRATROPIUM" EUROPEAN RESPIRATORY JOURNAL, MUNKSGAARD INTERNATIONAL PUBLISHERS, COPENHAGEN, DK, vol. 15, no. 5, May 2000 (2000-05), pages 878-885, XP001021107 ISSN: 0903-1936
- WESSELING G ET AL: "COMPARISON OF THE EFFECTS OF ANTICHOLINERGIC AND BETA2-AGONIST AND COMBINATION THERAPY ON RESPIRATORY IMPEDANCE IN COPD" CHEST, THE COLLEGE, CHICAGO, IL, US, vol. 101, no. 1, 1992, pages 166-173, XP000913861 ISSN: 0012-3692
- BACULARD A: "PLACE DU BRONCHODUAL DANS LE TRAITEMENT DE FOND DE L'ASTHME DE L'ENFANT BRONCHODUAL IN THE LONG-TERM TREATMENT OF CHILDREN WITH ASTHMA" ARCHIVES DE PEDIATRIE, ELSEVIER, PARIS, FR, vol. 2, no. SUPPL 2, 1995, pages 149S-153S, XP009010409 ISSN: 0929-693X
- BRIGGS D D ET AL: "Long-term pharmacologicmanagement of patients with chronic obstructive pulmonary disease" CLINICAL CORNERSTONE, EXCERPTA MEDICA,, GB, vol. 6, no. 2, 2004, pages S17-S28, XP004825197 ISSN: 1098-3597
- CAZZOLA ET AL: "Incremental Benefit of Adding Oxitropium Bromide to Formoterol in Patients with Stable COPD" PULMONARY PHARMACOLOGY AND THERAPEUTICS, ACADEMIC PRESS, NEW YORK, NY, US, vol. 12, 1999, pages 267-271, XP002210290 ISSN: 1094-5539

## Description

This invention relates to organic compounds and their use as pharmaceuticals, in particular for the treatment of inflammatory or obstructive airways diseases.

Cydulka et al, Annals of Emergency Medicine, volume 25, April 1995, pages 470-473 discloses treating patients that have acute exacerbation of chronic obstructive pulmonary disease with nebulised glycopyrrolate and nebulised albuterol.

Tzelepis et al, European Respiratory Journal, 1996, 9, pages 100-103 discloses treating patients that have chronic obstructive pulmonary disease with nebulised glycopyrrolate and nebulised metaproterenol.

In one aspect, the present invention provides a medicament comprising, separately or together, (A) glycopyrrolate and (B) (R)-5-[2-(5,6-diethyl-indan-2-ylamino)-1-hydroxy-ethyl]-8-hydroxy-1H-quinolin-2-one maleate, for simultaneous or sequential administration in the treatment of an inflammatory or obstructive airways disease.

In another aspect, the present invention provides a pharmaceutical composition comprising a mixture of effective amounts of (A) and (B), optionally together with at least one pharmaceutically acceptable carrier.

The invention further provides the use of (A) and (B) in the preparation of a medicament for combination therapy by simultaneous or sequential administration of (A) and (B) in the treatment of chronic obstructive pulmonary disease.

Preferably the molar ratio of (A) to (B) is from 100:1 to 1:300, for example 50:1 to 1:100, especially from 10:1 to 1:20, and more especially from 3:1 to 1:7.

(A) Glycopyrrolate is a known antimuscarinic agent. More specifically it inhibits acetyl choline binding to M3 muscarinic receptors thereby inhibiting bronchoconstriction.

Glycopyrrolate is a quaternary ammonium salt. Suitable counter ions are pharmaceutically acceptable counter ions including, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1-hydroxynaphthalene-2-carboxylate, 3-hydroxynaphthalene-2-carboxylate, methanesulfonate and benzenesulfonate. Its bromide salt, namely 3-[(cyclopentyl-hydroxyphenylacetyl)oxy]-1,1-dimethylpyrrolidinium bromide, has the following structural formula 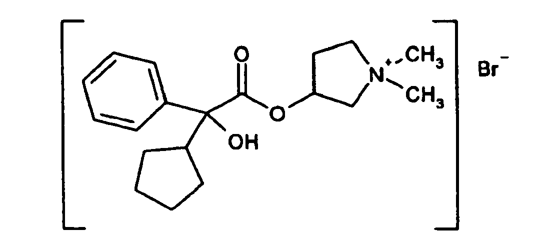 and can be prepared using the procedures described in United States patent US 2956062. _

Glycopyrrolate has two stereogenic centres and hence exists in four isomeric forms, namely (3R,2'R)-, (3S,2'R)-, (3R,2'S)- and (3S,2'S)-3-[(cyclopentyl-hydroxyphenylaceryl)oxy]-1,1-dimethylpyrrolidinium bromide, as described in United States patent specifications US 6307060 and US 6,613,795. The present invention embraces using one or more of these isomeric forms, especially the 3S,2'R isomer, the 3R,2'R isomer or the 2S,3'R isomer, thus including single enantiomers, or racemates, especially the (3S,2'R/2S,3'R) racemate.

(B) (R)-5-[2-(5,6-diethyl-indan-2-ylamino)-1-hydroxy-ethyl]-8-hydroxy-1H-quinolin-2-one maleate possesses beta-2 adrenoceptor agonist activity. It has a rapid onset of action and a prolonged stimulating action on the 2-adrenoceptor, for example up 24 hours or longer.

(R)-5-[2-(5,6-diethyl-indan-2-ylamino)-1-hydroxy-ethyl]-8-hydroxy-1H-quinolin-2-one maleate may be prepared by using the procedures described in international patent applications WO 2000/075114, WO 2003/076387, WO 2004/076422 or WO 2004/08766.

The medicament of the present invention may additionally contain one or more co-therapeutic agents such as anti-inflammatory, bronchodilatory, antihistamine, decongestant or anti-tussive drug substances, for example as potentiators of therapeutic activity of such drugs or as a means of reducing required dosaging or potential side effects of such drugs.

Co-therapeutic agents include steroids, A_{2A} agonists, A_{2B} antagonists, antihistamines, caspase inhibitors, LTB4 antagonists, LTD4 antagonists, PDE4 inhibitors, mucolytics, matrix metal loproteinase inhibitors (MMPi's), leukotrienes, antibiotics, anti neoplastics, peptides, vaccines, nicotine, elastase inhibitors and sodium cromoglycate.

Such anti-inflammatory drugs include steroids, for example glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920, and non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935, WO 04/26248, WO 0505452. Suitable A_{2A} agonists include those described in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618 and WO 04/046083. Suitable A_{2B} antagonists include those described in WO 03/042214 and WO 02/42298. Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in WO 03/099807, WO 04/026841, JP 2004107299. Suitable caspase inhibitors, including interleukin- I P converting enzyme (ICE) inhibitors, include those that are disclosed in Canadian patent specification 2109646, EP 519748, EP 547 699, EP 590 650, EP 628550, EP 644 197, EP 644198, WO 93/05071, WO 93/14777, WO 93/16710, WO 94/00154, WO 94/03480, WO 94/21673, WO 95/05152, WO 95/35308, WO 97/22618, WO 97/22619, WO 98/41232, WO 99/06367, WO 99/65451, WO 01/119373, US 5411985, US 5416013; US 5430128, US 5434248, US 5565430, US 5585357, US 5656627, US 5677283, US 6054487, US 6531474, US 20030096737, GB 2,278,276 as well as those disclosed in international patent applications WO 98/10778, WO 98/11109, WO 98/11129 and WO 03/32918. Suitable LTB4 antagonists include LY293111, CGS025019C, CP-195543, SC-53228, BIIL 284, ONO 4057, SB 209247 and those described in US 5451700 and WO 04/108720. Suitable LTD4 antagonists include montelukast and zafirlukast. Suitable PDE4 inhibitors PDE4 inhibitors such as cilomilast (Ariflo® GlaxoSmithKline), Roflumilast (Byk Gulden), V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), GRC 3886 (Glenmark), and those described in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839 and WO 04/005258, WO 04018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945, WO 04/045607, WO 04/037805, WO 04/063197, WO 04/103998 and WO 04/111044.

While (A) glycopyrrolate is an M3 antagonist, the medicament of the present invention optionally includes one or more other M3 antagonists such as ipratropium bromide, oxitropium bromide, tiotropium salt, CHF 4226 (Chiesi), or those described in WO 02/51841, WO 02/53564, WO 03/00840, WO 03/87094, WO 04/05285, WO 02/00652, WO 03/53966, EP 424021, US 5171744, US 3714357, WO 03/33495, WO 04/018422 or WO 05/003090.

While (B) is a beta-2 adrenoceptor agonist, the medicament of the present invention optionally includes one or more other beta-2 adrenoceptor agonists such as albuterol (salbutamol), metaproterenol, terbutaline, salmeterol fenoterol, procaterol, and especially, formoterol, carmoterol and pharmaceutically acceptable salts thereof, compounds (in free or salt or solvate form) of formula I of WO 04/087142, or those described in JP 05025045, US 2002/0055651, WO 93/18007, WO 99/64035, WO 01/42193, WO 01/83462, WO 02/066422, WO 02/070490, WO 02/076933, WO 03/24439, WO 03/72539, WO 03/42160, WO 03/91204, WO 03/42164, WO 03/99764, WO 04/11416, WO 04/16578, WO 04/22547, WO 04/32921, WO 04/33412, WO 04/37773, WO 04/37807, WO 04/39762, WO 04/39766, WO 04/45618, WO 04/46083, WO 04/80964, WO 04/108675 or WO 04/108676.

Administration of the medicament or pharmaceutical composition as hereinbefore described, i.e. with (A) and (B) in admixture or separate, is preferably by inhalation, i.e. (A) and (B) are in inhalable form. The inhalable form of the medicament may be, for example, an atomizable composition such as an aerosol comprising the active ingredient, i.e. (A) and (B) separately or in admixture, in solution or dispersion in a propellant, or a nebulizable composition comprising a solution or dispersion of the active ingredient in an aqueous, organic or aqueous/organic medium. For example, the inhalable form of the medicament may be an aerosol comprising a mixture of (A) and (B) in solution or dispersion in a propellant, or a combination of an aerosol containing (A) in solution or dispersion in a propellant with an aerosol containing (B) in solution or dispersion in a propellant. In another example, the inhalable form is a nebulizable composition comprising a dispersion of (A) and (B) in an aqueous, organic or aqueous/organic medium, or a combination of a dispersion of (A) in such a medium with a dispersion of (B) in such a medium.

An aerosol composition suitable for use as the inhalable form of the medicament may comprise the active ingredient in solution or dispersion in a propellant, which may be chosen from any of the propellants known in the art. Suitable such propellants include hydrocarbons such as n-propane, n-butane or isobutane or mixtures of two or more such hydrocarbons, and halogen-substituted hydrocarbons, for example chlorine and/or fluorine-substituted methanes, ethanes, propanes, butanes, cyclopropanes or cyclobutanes, such as dichlorodifluoromethane (CFC 12), trichlorofluoromethane (CFC11), 1,2-dichloro-1,1,2,2-tetrafluoroethane (CFC114) or, particularly, 1,1,1,2-tetrafluoroethane (HFA134a) and 1,1,1,2,3,3,3-heptafluoropropane (HFA227), or mixtures of two or more such halogen-substituted hydrocarbons. Where the active ingredient is present in suspension in the propellant, i.e. where it is present in particulate form dispersed in the propellant, the aerosol composition may also contain a lubricant and a surfactant, which may be chosen from those lubricants and surfactants known in the art. Other suitable aerosol compositions include surfactant-free or substantially surfactant-free aerosol compositions. The aerosol composition may contain up to about 5% by weight, for example 0.0001 to 5%, 0.001 to 5%, 0.001 to 3%, 0.001 to 2%, 0.001 to 1%, 0.001 to 0.1%, or 0.001 to 0.01% by weight of the active ingredient, based on the weight of the propellant. Where present, the lubricant and surfactant may be in an amount up to 5% and 0.5% respectively by weight of the aerosol composition. The aerosol composition may also contain a co-solvent such as ethanol in an amount up to 30% by weight of the composition, particularly for administration from a pressurised metered dose inhalation device. The aerosol composition may further contain a bulking agent, for example a sugar such as lactose, sucrose, dextrose, mannitol or sorbitol, in an amount, for example, of up to 20%, usually 0.001 to 1%, by weight of the composition.

In another embodiment of the invention, the inhalable form is a dry powder, i.e. (A) and (B) are present in a dry powder comprising finely divided (A) and (B) optionally together with at least one particulate pharmaceutically acceptable carrier, which may be one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. An especially preferred carrier is lactose. The dry powder may be contained as unit doses in capsules of, for example, gelatin or plastic, or in blisters (e.g. of aluminium or plastic), for use in a dry powder inhalation device, which may be a single dose or multiple dose device, preferably in dosage units of (A) and (B) together with the carrier in amounts to bring the total weight of powder per capsule to from 5 mg to 50 mg. Alternatively, the dry powder may be contained in a reservoir in a multi-dose dry powder inhalation device adapted to deliver, for example, 3-25mg of dry powder per actuation.

In the finely divided particulate form of the medicament, and in the aerosol composition where the active ingredient is present in particulate form, the active ingredient may have an average particle diameter of up to about 10 um, for example 0.1 to 5 µm, preferably 1 to 5 µm. The particulate carrier, where present, generally has a maximum particle diameter up to 300 µm, preferably up to 212 µm, and conveniently has a mean particle diameter of 40 to 100 µm, e.g. 50 to 75 µm. The particle size of the active ingredient, and that of a particulate carrier where present in dry powder compositions, can be reduced to the desired level by conventional methods, for example by grinding in an air-jet mill, ball mill or vibrator mill, sieving, microprecipitation, spray-drying, lyophilisation or controlled crystallisation from conventional solvents or from supercritical media.

The medicament may be a controlled release formulation comprising finely divided particles of (A) and (B) within a hydrophobic matrix material, e.g. comprising magnesium stearate, for example as described in international patent application WO 01/76575.

The inhalable medicament may be administered using an inhalation device suitable for the inhalable form, such devices being well known in the art. Accordingly, the invention also provides a pharmaceutical product comprising a medicament or pharmaceutical composition as hereinbefore described in inhalable form as hereinbefore described in association with one or more inhalation devices. In a further aspect, the invention provides an inhalation device, or a pack of two or more inhalation devices, containing a medicament or pharmaceutical composition as hereinbefore described in inhalable form as hereinbefore described.

Where the inhalable form of the active ingredient is an aerosol composition, the inhalation device may be an aerosol vial provided with a valve adapted to deliver a metered dose, such as 10 to 100 µl, e.g. 25 to 50 µl, of the composition, i.e. a device known as a metered dose inhaler. Suitable such aerosol vials and procedures for containing within them aerosol compositions under pressure are well known to those skilled in the art of inhalation therapy. For example, an aerosol composition may be administered from a coated can, for example as described in EP 0642992 A.

Where the inhalable form of the active ingredient is a nebulizable aqueous, organic or aqueous/organic dispersion, the inhalation device may be a known nebulizer, for example a conventional pneumatic nebulizer such as an airjet nebulizer, or an ultrasonic nebulizer, which may contain, for example, from 1 to 50 ml, commonly 1 to 10 ml, of the dispersion; or a hand-held nebulizer, sometimes referred to as a soft mist or soft spray inhaler, for example an electronically controlled device such as an AERx (Aradigm, US) or Aerodose (Aerogen), or a mechanical device such as a RESPIMAT (Boehringer Ingelheim) nebulizer which allows much smaller nebulized volumes, e.g. 10 to 100 µl, than conventional nebulizers.

Where the inhalable form of the active ingredient is the finely divided particulate form, the inhalation device may be, for example, a dry powder inhalation device adapted to deliver dry powder from a capsule or blister containing a dry powder comprising a dosage unit of (A) and (B) or a multidose dry powder inhalation (MDDPI) device adapted to deliver, for example, 3-25 mg of dry powder comprising a dosage unit of (A) and (B) per actuation. The dry powder formulation preferably contains the active ingredients optionally together with a diluent or carrier, such as lactose, of the desired particle size distribution and a compound that helps to protect against product performance deterioration due to moisture e.g. magnesium stearate. Suitable such dry powder inhalation devices are well known. For example, a suitable device for delivery of dry powder in encapsulated form is that described in US 3991761, while a suitable MDPI device is that described in WO 97/20589.

The medicament of the invention is preferably a pharmaceutical composition comprising a mixture of (A) as hereinbefore defined and (B) as hereinbefore defined, preferably together with at least one pharmaceutically acceptable carrier as hereinbefore described.

The molar ratio of (A) to (B) may be, in general, from 100:1 to 1:300, for example from 50:1 to 1:100 or from 20:1 to 1:50, preferably from 10:1 to 1:20, more preferably from 5:1 to 1:10, from 3:1 to 1:7 or from 2:1 to 1:2. The compound (A) and the compound (B) may be administered separately in the same ratio.

A suitable daily dose of the compound (A), particularly as the bromide salt, for inhalation may be from 10 µg to 2000 µg, preferably from 60 to 1000 µg, and especially from 80 to 800 µg, e.g. from 20 to 500 µg.

A suitable daily dose of compound (B) for inhalation may be from 10 µg to 2000 µg, for example from 10 to 1500 µg, from 10 to 1000 µg, preferably from 20 to 800 µg, e.g. from 20 to 600 µg or from 20 to 500 µg.

A suitable unit dose of compound (A), particularly as the bromide salt, may be from 10 µg to 2000 µg, preferably from 60 to 1000 µg, especially from 80 to 800 µg, e.g. from 20 to 500 µg.

A suitable unit dose of compound (B) may be from 10 µg to 2000 µg, for example from 10 to 1500 µg, from 10 to 1000 µg, preferably from 20 to 800 µg, e.g. from 20 to 600 µg or from 20 to 500 µg.

These unit doses may be administered once or twice daily in accordance with the daily doses mentioned hereinbefore. A single dose is preferred. The precise unit and daily dose used will of course depend on the condition to be treated, the patient and the efficiency of the inhalation device.

In one preferred embodiment of the invention, the medicament of the invention is a pharmaceutical composition which is a dry powder in a capsule containing a unit dose of (A) and (B), for example for inhalation from a single capsule inhaler, the capsule suitably containing a unit dose of (A) e.g. as hereinbefore described, and a unit dose of (B), e.g. as hereinbefore described, together with a pharmaceutically acceptable carrier as hereinbefore described in an amount to bring the total weight of dry powder per capsule to between 5 mg and 50 mg, for example 5 mg, 10 mg, 15 mg, 20 mg, 25 mg, 30 mg, 35 mg, 40 mg, 45 mg or 50 mg.

In a further preferred embodiment of the invention, the medicament of the invention is a pharmaceutical composition which is an aerosol comprising (A) and (B) e.g. in a ratio as hereinbefore described, in a propellant as hereinbefore described, optionally together with a surfactant and/or a bulking agent and/or a co-solvent such as ethanol as hereinbefore described, for administration from a metered dose inhaler adapted to deliver an amount of aerosol containing a unit dose of (A) and a unit dose of (B), or a known fraction of a unit dose of (A) and a known fraction of a unit dose of (B), per actuation. Thus if, for example, the inhaler delivers half of the unit doses of (A) and (B) per actuation, the unit doses can be administered by two actuations of the inhaler.

In accordance with the above, the invention also provides a pharmaceutical kit comprising (A) and (B) as hereinbefore defined in separate unit dosage forms, said forms being suitable for administration of (A) and (B) in effective amounts. Such a kit suitably further comprises one or more inhalation devices for administration of (A) and (B). For example, the kit may comprise one or more dry powder inhalation devices adapted to deliver dry powder from a capsule, together with capsules containing a dry powder comprising a dosage unit of (A) and capsules containing a dry powder comprising a dosage unit of (B). In another example, the kit may comprise a multidose dry powder inhalation device containing in the reservoir thereof a dry powder comprising (A) and a multidose dry powder inhalation device containing in the reservoir thereof a dry powder comprising (B). In a further example, the kit may comprise a metered dose inhaler containing an aerosol comprising (A) in a propellant and a metered dose inhaler containing an aerosol comprising (B) in a propellant.

The medicament of the invention is advantageous in the treatment of inflammatory or obstructive airways disease, exhibiting highly effective bronchodilatory and anti-inflammatory properties. For instance, it is possible using the combination therapy of the invention to reduce the dosages of (A) or (B) required for a given therapeutic effect compared with those required using treatment with either (A) or (B) alone, thereby minimising possibly undesirable side effects. Furthermore, using the combinations of the invention, particularly using compositions containing (A) and (B), medicaments which have a rapid onset of action and a long duration of action may be prepared. Moreover, using such combination therapy, medicaments which result in a significant improvement in lung function may be prepared. In another aspect, using the combination therapy of the invention, medicaments which provide effective control of obstructive or inflammatory airways diseases, or a reduction in exacerbations of such diseases, may be prepared. In a further aspect, using compositions of the invention containing (A) and (B), medicaments which reduce or eliminate the need for treatment with short-acting rescue medicaments such as salbutamol or terbutaline, may be prepared; thus compositions of the invention containing (A) and (B) facilitate the treatment of an obstructive or inflammatory airways disease with a single medicament.

Treatment of inflammatory or obstructive airways diseases in accordance with the invention may be symptomatic or prophylactic treatment. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy

Other inflammatory or obstructive airways diseases and conditions to which the present invention is applicable include acute/adult lung injury (ALI), adult/acute respiratory distress syndrome (ARDS), cystic fibrosis, chronic obstructive pulmonary, airways or lung disease (COPD, COAD or COLD), including chronic bronchitis and emphysema, bronchiectasis and exacerbation of airways hyperreactivity consequent to other drug therapy, in particular other inhaled drug therapy. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tobacosis and byssinosis.

The invention is illustrated by the following Examples.

### EXAMPLES

### Compound A1

**3-[(cyclopentyl-hydroxyphenylacetyl)oxy]-1,1-dimethylpyrrolidinium bromide (glycopyrrolate)** This compound is commercially available as a racemate or is prepared using the procedures described in United States patent US 2956062.

### Compound B1

**(R)-5-[2-(5,6-diethyl-indan-2-ylamino)-1-hydroxy-ethyl]-8-hydroxy-1H-quinolin-2-one maleate** This compound is prepared using the procedures described in international patent applications WO 2000/075114.

Gelatin capsules suitable for use in a capsule inhaler such as that described in US 3991761 and EP 1270034 are prepared, each capsule containing a dry powder obtained by mixing Compound A1 and Compound B1 which have been ground to a mean particle diameter of 1 to 5 µm and lactose monohydrate having a particle diameter below 212 µm, the amounts being as shown in the Table 1 below:

**TABLE 1**

| **Example** | **Compound A1** | **Compound B1** | **Lactose** |
|---|---|---|---|
| | **(Parts)** | **(Parts)** | **(Parts)** |
| 1 | 20 | 100 | 19880 |
| 2 | 40 | 100 | 19860 |
| 3 | 80 | 100 | 19820 |
| 4 | 100 | 100 | 19800 |
| 5 | 120 | 100 | 19780 |
| 6 | 140 | 100 | 19760 |
| 7 | 160 | 100 | 19740 |
| 8 | 180 | 100 | 19720 |
| 9 | 200 | 100 | 19700 |
| 10 | 220 | 100 | 19680 |
| 11 | 240 | 100 | 19660 |
| 12 | 300 | 100 | 19600 |
| 13 | 500 | 100 | 19400 |
| 14 | 1000 | 100 | 18900 |
| 15 | 2000 | 100 | 17900 |
| 16 | 20 | 100 | 24880 |
| 17 | 40 | 100 | 24860 |
| 18 | 80 | 100 | 24820 |
| 19 | 100 | 100 | 24800 |
| 20 | 120 | 100 | 24780 |
| 21 | 140 | 100 | 24760 |
| 22 | 160 | 100 | 24740 |
| 23 | 180 | 100 | 24720 |
| 24 | 200 | 100 | 24700 |
| 25 | 220 | 100 | 24680 |
| 26 | 240 | 100 | 24660 |
| 27 | 300 | 100 | 24600 |
| 28 | 500 | 100 | 24400 |
| 29 | 1000 | 100 | 23900 |
| 30 | 2000 | 100 | 22900 |
| 31 | 20 | 200 | 14780 |
| 32 | 40 | 200 | 14760 |
| 33 | 80 | 200 | 14720 |
| 34 | 100 | 200 | 14700 |
| 35 | 120 | 200 | 14680 |
| 36 | 140 | 200 | 14660 |
| 37 | 160 | 200 | 14640 |
| 38 | 180 | 200 | 14620 |
| 39 | 200 | 200 | 14600 |
| 40 | 220 | 200 | 14580 |
| 41 | 240 | 200 | 14560 |
| 42 | 300 | 200 | 14500 |
| 43 | 500 | 200 | 14300 |
| 44 | 1000 | 200 | 13800 |
| 45 | 2000 | 200 | 12800 |
| 46 | 20 | 200 | 24780 |
| 47 | 40 | 200 | 24760 |
| 48 | 80 | 200 | 24720 |
| 49 | 100 | 200 | 24700 |
| 50 | 120 | 200 | 24680 |
| 51 | 140 | 200 | 24660 |
| 52 | 160 | 200 | 24640 |
| 53 | 180 | 200 | 24620 |
| 54 | 200 | 200 | 24600 |
| 55 | 220 | 200 | 24580 |
| 56 | 240 | 200 | 24560 |
| 57 | 300 | 200 | 24500 |
| 58 | 500 | 200 | 24300 |
| 59 | 1000 | 200 | 23800 |
| 60 | 2000 | 200 | 22800 |

## Claims

1. A medicament comprising, separately or together, (A) glycopyrrolate and (B) (R)-5-[2-(5,6-diethyl-indan-2-ylamino)-1-hydroxy-ethyl]- 8-hydroxy-1H-quinolin-2-one maleate, for simultaneous or sequential administration in the treatment of an inflammatory or obstructive airways disease.

2. A medicament according to claim 1 wherein glycopyrrolate is a racemate.

3. A medicament according to claim 1 wherein glycopyrrolate is a single enantiomer.

4. A medicament according to claim 3 wherein glycopyrrolate is (3S,2'R)-3-[(cyclopentylhydroxyphenylacetyl)oxy]-1,1-dimethylpyrrolidinium bromide or (3R,2'R)-3-[(cyclopentylhydroxyphenylacetyl)oxy]-1,1-dimethylpyrrolidinium bromide.

5. A medicament according to any preceding claim that further contains at least one drug substance which is an anti-inflammatory, a bronchodilator, an antihistamine, a decongestant or an anti-tussive drug substance.

6. A medicament according to any one of claims 1 to 4, which is in inhalable form and is
(i) an aerosol comprising a mixture of (A) and (B) in solution or dispersion in a propellant; or
(ii) a combination of an aerosol containing (A) in solution or dispersion in a propellant with an aerosol containing (B) in solution or dispersion in a propellant; or
(iii) a nebulizable composition comprising a dispersion of (A) and (B) in an aqueous, organic or aqueous/organic medium; or
(iv) a combination of a dispersion of (A) in an aqueous, organic or aqueous/organic medium with a dispersion of (B) in an aqueous, organic or aqueous/organic medium.

7. A medicament according to any one of claims 1 to 4, in which (A) and/or (B) are present in inhalable form as a dry powder comprising finely divided (A) and/or (B) optionally together with at least one particulate pharmaceutically acceptable carrier.

8. A medicament according to claim 1 which is a pharmaceutical composition comprising a mixture of effective amounts of (A) and (B) optionally together with at least one pharmaceutically acceptable carrier.

9. A medicament according to any preceding claim, in which the molar ratio of (A) to (B) is from 100:1 to 1:300.

10. A medicament according to claim 9, in which the molar ratio of (A) to (B) is from 50:1 to 1:100.

11. A medicament according to any claim 10, in which the molar ratio of (A) to (B) is from 10:1 to 1:20.

12. A medicament according to claim 8, which is
a dry powder in a capsule, the capsule containing a unit dose of (A), a unit dose of (B) and a pharmaceutically acceptable carrier in an amount to bring the total weight of dry powder per capsule to between 5 mg and 50 mg; or
an aerosol comprising (A) and (B) in a ratio as hereinbefore specified in claim 9, in a propellant, optionally together with a surfactant and/or a bulking agent and/or a co-solvent suitable for administration from a metered dose inhaler adapted to deliver an amount of aerosol containing a unit dose of (A) and a unit dose of (B), or a known fraction of a unit dose of (A) and a known fraction of a unit dose of (B), per actuation.

13. A pharmaceutical kit comprising (A) and (B) as defined in any one of claims 1 to 4 in separate unit dosage forms, said forms being suitable for administration of (A) and (B) in effective amounts, together with one or more inhalation devices for administration of (A) and (B).

## Patentansprüche

1. Medikament, das getrennt oder zusammen (A) Glykopyrrolat und (B) (R)-5-[2-(5,6-Diethyl-indan-2-ylamino)-1-hydroxy-ethyl]-8-hydroxy-1H-chinolin-2-on-maleat umfasst, zur gleichzeitigen oder aufeinanderfolgenden Verabreichung bei der Behandlung einer entzündlichen oder obstruktiven Atemwegserkrankung.

2. Medikament nach Anspruch 1, wobei das Glykopyrrolat ein Racemat ist.

3. Medikament nach Anspruch 1, wobei das Glykopyrrolat ein einzelnes Enantiomer ist.

4. Medikament nach Anspruch 3, wobei das Glykopyrrolat (3S,2'R)-3-[(Cyclopentylhydroxyphenylacetyl)oxy]-1,1-dimethylpyrrolidiniumbromid oder (3R,2'R)-3-[(Cyclopentylhydroxyphenylacetyl)oxy]-1,1-dimethylpyrrolidiniumbromid ist.

5. Medikament nach einem der vorhergehenden Ansprüche, das ferner mindestens eine Arzneimittelsubstanz enthält, die eine entzündungshemmende, bronchienerweiternde, Antihistamin-, abschwellende oder hustenstillende Arzneimittelsubstanz ist.

6. Medikament nach einem der Ansprüche 1 bis 4, das in inhalierbarer Form vorliegt und
i) ein Aerosol, das ein Gemisch von (A) und (B) in Lösung oder Dispersion in einem Treibmittel umfasst; oder
ii) eine Kombination eines Aerosols, das (A) in Lösung oder Dispersion in einem Treibmittel enthält, mit einem Aerosol, das (B) in Lösung oder Dispersion in einem Treibmittel enthält, oder
iii) eine zerstäubbare Zusammensetzung, die eine Dispersion von (A) und (B) in einem wässrigen, organischen oder wässrigen/organischen Medium umfasst, oder
iv) eine Kombinaiton einer Dispersion von (A) in einem wässrigen, organischen oder wässrigen/organischen Medium mit einer Disperision von (B) in einem wässrigen, organischen oder wässrigen/organischen Medium ist.

7. Medikament nach einem der Ansprüche 1 bis 4, worin (A) und/oder (B) in inhalierbarer Form als trockenes Pulver vorhanden sind, das feinteiliges (A) und/oder (B) optional zusammen mit mindestens einem teilchenförmigen pharmazeutisch akzeptablen Träger umfasst.

8. Medikament nach Anspruch 1, bei dem es sich um eine pharmazeutische Zusammensetzung handelt, die ein Gemisch von wirksamen Mengen von (A) und (B) optional zusammen mit mindestens einem pharmazeutisch akzeptablen Träger umfasst.

9. Medikament nach einem der vorhergehenden Ansprüche, worin das Molverhältnis von (A) zu (B) 100:1 bis 1:300 beträgt.

10. Medikament nach Anspruch 9, worin das Molverhältnis von (A) zu (B) 50:1 bis 1:100 beträgt.

11. Medikament nach Anspruch 10, worin das Molverhältnis von (A) zu (B) 10:1 bis 1:20 beträgt.

12. Medikament nach Anspruch 8, bei dem es sich um
ein trockenes Pulver in einer Kapsel, wobei die Kapsel eine Einheitsdosis von (A), eine Einheitsdosis von (B) und einen pharmazeutisch akzeptablen Träger in einer Menge, um das Gesamtgewicht des trockenen Pulvers pro Kapsel auf zwischen 5 mg und 50 mg zu bringen, enthält; oder
ein Aerosol handelt, das (A) und (B) in einem Verhältnis gemäß obiger Angabe in Anspruch 9 in einem Treibmittel, optional zusammen mit einem grenzflächenaktiven Mittel und/oder einem Füllstoff und/oder einem Co-Lösemittel mit Eignung zur Verabreichung aus einer Dosierinhaliervorrichtung, die darauf angepasst ist, eine Menge eines Aerosols, das eine Einheitsdosis von (A) und eine Einheitsdosis von (B) oder einen bekannten Anteil einer Einheitsdosis von (A) und einen bekannten Anteil einer Einheitsdosis von (B) enthält, pro Auslösung abzugeben, umfasst.

13. Pharmazeutisches Kit, das (A) und (B) gemäß Definition in einem der Ansprüche 1 bis 4 in getrennten Einheitsdosierungsformen umfasst, wobei die Formen für eine Verabreichung von (A) und (B) in wirksamen Mengen zusammen mit einer oder mehreren Inhalationsvorrichtungen zur Verabreichung von (A) und (B) geeignet sind.

## Revendications

1. Médicament comprenant, séparément ou conjointement, (A) du glycopyrrolate et (B) du maléate de (R)-5-[2-(5,6-diéthyl-indan-2-ylamino)-1-hydroxy-éthyl]-8-hydroxy-1H-quinoléin-2-one, pour administration simultanée ou successive dans le traitement d'une maladie inflammatoire ou obstructive des voies aériennes.

2. Médicament selon la revendication 1, dans lequel le glycopyrrolate est un racémate.

3. Médicament selon la revendication 1, dans lequel le glycopyrrolate est un énantiomère unique.

4. Médicament selon la revendication 3, dans lequel le glycopyrrolate est du bromure de (3S,2'R)-3-[(cyclopentyl-hydroxyphénylacétyl)oxy]-1,1-diméthylpyrrolidinium ou du bromure de (3R,2'R)-3-[(cyclopentylhydroxyphénylacétyl)oxy]-1,1-diméthylpyrrolidinium.

5. Médicament selon l'une quelconque des revendications précédentes, qui contient en outre au moins une substance médicamenteuse qui est une substance médicamenteuse anti-inflammatoire, bronchodilatatrice, antihistaminique, décongestionnante ou antitussive.

6. Médicament selon l'une quelconque des revendications 1 à 4, qui se présente sous forme inhalable et est
(i) un aérosol comprenant un mélange de (A) et de (B) en solution ou en dispersion dans un gaz propulseur ; ou
(ii) une combinaison d'un aérosol contenant (A) en solution ou en dispersion dans un gaz propulseur avec un aérosol contenant (B) en solution ou en dispersion dans un gaz propulseur ; ou
(iii) une composition nébulisable comprenant une dispersion de (A) et de (B) dans un milieu aqueux, organique ou aqueux/organique ; ou
(iv) une combinaison d'une dispersion de (A) dans un milieu aqueux, organique ou aqueux/organique avec une dispersion de (B) dans un milieu aqueux, organique ou aqueux/organique.

7. Médicament selon l'une quelconque des revendications 1 à 4, dans lequel (A) et/ou (B) sont présents sous forme inhalable sous la forme d'une poudre sèche comprenant (A) et/ou (B) finement divisés éventuellement conjointement avec au moins un véhicule particulaire pharmaceutiquement acceptable.

8. Médicament selon la revendication 1, qui est une composition pharmaceutique comprenant un mélange de quantités efficaces de (A) et de (B) éventuellement conjointement avec au moins un véhicule pharmaceutiquement acceptable.

9. Médicament selon l'une quelconque des revendications précédentes, dans lequel le rapport molaire de (A) à (B) est compris entre 100:1 et 1:300.

10. Médicament selon la revendication 9, dans lequel le rapport molaire de (A) à (B) est compris entre 50:1 et 1:100.

11. Médicament selon la revendication 10, dans lequel le rapport molaire de (A) à (B) est compris entre 10:1 et 1:20.

12. Médicament selon la revendication 8, qui est une poudre sèche dans une capsule, la capsule contenant une dose unitaire de (A), une dose unitaire de (B) et un véhicule pharmaceutiquement acceptable en une quantité propre à porter le poids total de poudre sèche par capsule entre 5 mg et 50 mg ; ou
un aérosol comprenant (A) et (B) en un rapport tel que spécifié ci-dessus dans la revendication 9, dans un gaz propulseur, éventuellement conjointement avec un agent tensio-actif et/ou un agent de charge et/ou un co-solvant appropriés à l'administration à partir d'un inhalateur doseur adapté pour délivrer une certaine quantité d'aérosol contenant une dose unitaire de (A) et une dose unitaire de (B), ou une fraction connue d'une dose unitaire de (A) et une fraction connue d'une dose unitaire de (B), par actionnement.

13. Trousse pharmaceutique comprenant du (A) et du (B) tels que définis dans l'une quelconque des revendications 1 à 4, sous des formes galéniques unitaires séparées, lesdites formes étant appropriées à l'administration de (A) et de (B) en des quantités efficaces, conjointement avec un ou plusieurs dispositifs d'inhalation pour l'administration de (A) et de (B).
